(19) Europäisches Patentamt
European Patent Office
Office européen des brevets

(11) EP 0 800 345 B1

(12) EUROPEAN PATENT SPECIFICATION

(45) Date of publication and mention of the grant of the patent:
**03.04.2002 Bulletin 2002/14**

(21) Application number: **95944684.0**

(22) Date of filing: **29.12.1995**

(51) Int Cl.[7]: **A01N 35/02**

(86) International application number:
**PCT/US95/17049**

(87) International publication number:
**WO 96/20595 (11.07.1996 Gazette 1996/31)**

# (54) TOXIC METABOLITE MODULATION IN CONSUMABLE PRODUCTS

REGULIERUNG DES GEHALTES TOXISCHER METABOLITEN IN NAHRUNGSMITTELN

MODULATION DU NIVEAU DES METABOLITES TOXIQUES DANS LES PRODUITS DE CONSOMMATION

(84) Designated Contracting States:
**AT BE CH DE DK ES FR GB GR IE IT LI LU MC NL PT SE**

(30) Priority: **30.12.1994 US 367082**
**07.06.1995 US 485035**

(43) Date of publication of application:
**15.10.1997 Bulletin 1997/42**

(60) Divisional application:
**99203496.7 / 1 000 543**

(73) Proprietor: **PROGUARD, INC.**
**Suisun City, CA 94585 (US)**

(72) Inventors:
- **EMERSON, Ralph, W.**
  **Davis, CA 95616 (US)**
- **CRANDALL, Bradford, G., Jr.**
  **Davis, CA 95616 (US)**

(74) Representative:
**Smulders, Theodorus A.H.J., Ir. et al**
**Vereenigde**
**Postbus 87930**
**2508 DH Den Haag (NL)**

(56) References cited:
**EP-A- 0 383 430** **FR-A- 2 529 755**
**US-A- 4 978 686**

- **BRIGHTON CROP PROT. CONF.-PESTS AND DISEASES, no. 1, 1994, pages 307-312, XP002002079 L.G.M.GORRIS, K.OOSTERHAVEN, K.J.HARTMANS, Y.DE WITTE & E.J. SMID: "Control of fungal storage diseases of potato by use of plant essential oil components."**
- **DATABASE WPI Section Ch, Week 9327 Derwent Publications Ltd., London, GB; Class C05, AN 93-216621 XP002002081 & JP,A,05 139 924 (ITOEN KK) , 8 June 1993**
- **LETT. APPL. MICROBIOL., vol. 19, no. 2, 1994, pages 110-113, XP002002080 A.-L.E. MAHMOUD: "Antifungal action and antiaflatoxigenic properties of some essential oil constituents."**
- **DATABASE WPI Section Ch, Week 8620 Derwent Publications Ltd., London, GB; Class C03, AN 86-128179 XP002002082 & JP,A,61 065 802 (MARUZEN KASEI KK) , 4 April 1986**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

## INTRODUCTION

### Field of the Invention

The invention relates to methods for controlling the level of toxic metabolites present in consumable products. The compositions used in these methods include aromatic aldehydes.

### Background of the Invention

Mold-induced contamination and deterioration of agricultural products causes economic loss and poses health hazards. Fungi from the genera *Aspergillus, Alternaria*, *Fusarium*, and *Penicillium* can contaminate food crops and their products. Mycotoxins produced by these fungi include aflatoxins, fumonasins, fusaric acid, TA/AAL toxins, zearalenone, and trichothecene, 5-butylpicolinic acid and related phytotoxic pyridine derivatives. These mycotoxins are highly toxic to a variety of species including humans and can be found in commercially prepared food stuffs and animal feed. Serious health problems may arise when fowl, fish, animals or humans ingest materials that have been contaminated with certain genera of fungi which produce mycotoxins.

Mycotoxins are secondary metabolites; those produced by various species of *Aspergillus* are the best known. As an example. *Aspergillus flavus* grows on a variety of plant materials and produces a low molecular weight mycotoxin, aflatoxin, that is poisonous to humans and many other animal species. The real and potential danger of these toxins was dramatically shown in 1960 by a large-scale trout poisoning in commercial fish hatcheries caused by fish food contaminated by fungi. The fumonisins also have been shown to affect the growth and health of domesticated animals of commercial value including horses, pigs, chickens and turkeys. Fumonisins also have been identified in food stuffs used to feed animals employed broadly in biomedical research and commercial testing. Research therefore may be compromised when experimental animals are reared on food variably contaminated with mycotoxins. Fumonisin represents a class of mycotoxins which are contaminants found in certain food crop products. Also, the same Fusarium fungi which colonize stored corn and corn products produce fusaric acid. The Fusarium mycotoxins are heat stable and survives extensive processing and is found in many corn products, including fructose corn sweetener, the major source of sweeteners in the food industry, and commercially produced fermentation products such as grain alcohol. Other mycotoxins which have been identified in corn materials and other vegetable material include, TA/AAL toxins (structure analogs of fumonisin), aflatoxins, zearalenone, trichothecene, 5-butylpicolinic acid and related phytotoxic pyridine derivatives.

Fusaric acid has been shown to affect neurotransmitters, therefore nerve function of both the central and peripheral nervous systems and heart function may be affected upon exposure to fusaric acid and fumonisin-contaminated materials and products. Thus, elimination of fusaric acid and other mycotoxins that have yet to be identified from consumable materials may play a significant part in the well-being of animals and humans alike.

The presence of mycotoxins in food is attributable to the wide-spread distribution of fungi and their growth during storage and handling of contaminated food and crops. High levels of all types of mycotoxins have been found in a variety of edible commodities including beans, cereals, coconuts, peanuts, sweet potatoes and commercially prepared animal feeds. Mycotoxins also have been identified in milk and milk products. Outside of using chemical preservatives, pasteurization or tightly controlled and dry storage conditions, steps to prevent or reduce the level of mycotoxins present in these commercial products have proved ineffective.

Mycotoxin contamination of tobacco products may be linked to the pathology of diseases associated with tobacco consumption. Tobacco plants are often severely contaminated with fungi that produce mycotoxins, including the fumonisin and fusaric acid producing fungi. Fumonisins have been implicated in esophageal cancer in humans, the third most frequent cancer world-wide. Both fumonisin and fusaric acid producing organisms are present on tobacco and thus may play an important, and heretofore unrecognized role among consumers of both cigarette and smokeless tobacco, in oral, throat, and lung cancers, as well as other diseases associated with tobacco consumption. Tobacco also has been shown to be contaminated with other mycotoxins including the aflatoxins.

Chemically, mycotoxins are heat stable compounds of relatively low molecular weight. Thus, once food or other consumables become contaminated with unacceptably high levels of mycotoxin, the product generally must be discarded. It therefore is of interest to develop methods that can be used to prevent contamination of consumable, and to identify the presence of mycotoxins in a product, to detoxify toxin-contaminated non-consumable materials to decrease the negative health affects associated with exposure to these materials. Moreover, it is highly desirable to control the level and toxic effects of microbial toxins that accumulate before, during and/or after consumable products enter the food chain or are otherwise consumed.

Relevant Literature

The presence of mycotoxins in stored leaves of chewing tobacco is described in Varma *et al., Mycopathologia* (1991), 113:19-23. The antibotulinal properties of various aromatic and aliphatic aldehyde are disclosed in Bowles and Miller, *J. Food Protection* (1993) 56:788-794. Other formulations which include cinnamic aldehyde have been reported to protect crops from attack by pathogenic microbes. *See* U.S. Patent Nos. 4,978,686 and 5,149,715 and French patent application 2529755. Film-forming and/or antitranspirant coating polymers such as sodium bicarbonate and light paraffinic petroleum oils have been reported to control the level of fungal colonization. Horst *et al.* (*Plant Disease,* March 1992, p.247), Elad *et al.* (*Phytoparasitica* (1989) 17: 279-288) and Hagiladi *et al.* (*J. Environ. Hortic.* (1986) 4: 69-71). Protection of farm products from insects, microbes and bacteria to prevent physical harm to the farm products using an emulsion of cinnamic aldehyde is reported in Japanese patent application 814965. The formulation is reported to be rapid acting and to leave no residue.

Gorris *et al.* in the Brighton Crop Prot. Conf. -pests and diseases no. 1, 1994, pages 307-312, disclose the use of extracts of essential oils or purified compounds of essential oils to suppress growth of fungal storage pathogens of potato in vitro. The compositions disclosed consist essentially of oils, which are phytotoxic and further does not disclose or suggest a surfactant.

US-A 4 978 686 discloses a method for the protection of crops from insect pests, micro-organisms and pathogenic micro-organisms by applying to the crops a compositions comprising cinnamic aldehyde and an antioxidant and optionally an emulsifier. Further, the compositions according to US-A 4 978 686 only provide a short term efficacy.

FR-A 2 259 755 describes the use of cinnamic aldehyde to protect crops against parasitic mushrooms, molds, harmful insects, bacteria and other parasitic predators. The publication further discloses that additional agricultural products can prolong the fungicidal effect of cinnamic aldehyde but does not show any bio-control of micro-organisms nor provides a teaching on how to provide for a long term protective bio-control effect of cinnamic aldehyde.

## SUMMARY OF THE INVENTION

The present invention relates to methods and compositions for controlling the level of toxic metabolites present in a variety of consumable products that are either colonized or capable of being colonized by toxin-producing micro-organisms. The method includes the steps of contacting a consumable product or a precursor of such product with a composition, particularly a an aromatic aldehyde which limits the colonization of, kills or displaces one or more micro-organisms which colonize the consumable material or precursor and which produce toxin(s). The invention finds use in controlling the level of toxic metabolites present in consumable products derived from plant materials, as well as decreasing contamination of the food chain by fungal toxins and toxic metabolites.

More in detail, the present invention relates to a method for biocontrol of toxin-producing micro-organisms on a harvested plant part, said method comprising:
providing a plant part prior to or after harvesting of said plant part with an effective microbial growth modulating amount of a formulation comprising saponin or polyoxyethylene(20)sorbitan mono oleate and 0.01 g/l to 25 g/l of at least one of cinnamic aldehyde, coniferyl aldehyde or $\alpha$-hexyl cinnamic aldehyde, wherein said formulation is non-phytotoxic to said plant and no other antioxidants than of cinnamic aldehyde, coniferyl aldehyde or $\alpha$-hexyl cinnamic aldehyde are provided; whereby micro-organisms on said plant part are biocontrolled.

## BRIEF DESCRIPTION OF THE PREFERRED EMBODIMENTS

Methods and compositions are provided for modulating the level of one or more toxic metabolites associated with consumable products, particularly agricultural products that are capable of being colonized by one or more toxin-producing microorganisms. The methods involve killing or long term displacement of one or more toxin producing microorganisms, particularly using the naturally occurring compounds, the aromatic aldehydes cinnamic aldehyde, coniferyl aldehyde and alpha hexyl cinnamic aldehyde (HCA). The invention is particularly suited for reducing the level of mycotoxins and other toxic secondary metabolites associated with plant parts such as stems, leaves, roots, fruit, seeds, and/or flowers before, during and/or after the plant and/or plant part is harvested and/or processed for consumption.

The invention offers several advantages over currently available detoxification techniques. One advantage is that contamination of consumable agricultural products can be prevented or significantly decreased to a level safe for consumption. Agricultural products can be treated either pre-harvest or post-harvest generally by way of a single application of the composition. Moreover, by treating a plant in the field with a substance which kills or displaces mycotoxin-producing fungi, the levels of toxin contamination can be significantly reduced in the harvested material. The aromatic aldehydes in particular have positive organoleptic and olfactory properties which in some cases may improve the flavor and/or smell of treated products. The odor of HCA for example is described as floral or jasmine-like

with some herbaceous character (Technical Data Sheet).

The aromatic aldehydes which may find use in the subject invention, such as cinnamaldehyde, are generally regarded as safe (GRAS) synthetic flavoring agents (21 CFR §172.515). HCA was in public use before the 1950's and today is widely used in consumer preparations (soaps, detergents, creams, lotions, perfumes) (Monographs on fragrances raw materials. Food Cosmet. Toxicol. 12: suppl., 915, 1974). HCA was granted GRAS (generally recognized as safe) status by FEMA (Flavoring Extract Manufacturers' Association. Survey of flavoring ingredient usage levels. No. 2569. Fd. Technol., Champaign, 19: (part 2) 155, 1965) in 1965 and is approved by the US FDA for use in food (21CFR121.1164). The Council of Europe (1970) (Council of Europe. Natural and Artificial Flavoring Substances. Partial Agreement in the Social and Public Health Field. Strasbourg, List A(1), Series 1, no. 129, p. 55, 1970) included HCA in the list of admissible artificial flavoring substances at a level of 1 ppm. Various of these compounds have been reported to have inhibitory activity against *C. botulinum* spore germination. Bowles and Miller, *G. Food Protection* (1993) 56: 788-794.

Surfactants which can be used as emulsifiers such as the Tweens (polysorbates) also already are used as food additives, as is saponin (which also has GRAS status). In addition, formulation residuality can be managed. This will be of great benefit when short term residuals are desired for integrated pest management programs with beneficial insects. In addition, the formulations work against pests which are resistant to other agents and are effective on multiple target organisms, including not only mycotoxin producing fungi but also insect targets. This reduces the need for application of multiple agents to the consumable material of interest. The effects of a single application of the formulation are long lasting, and generally a single application is sufficient to control fungal growth for at least a month or in some cases up to an entire growing season. The long term control of pathogenic organisms results in a healthier plant and an improved yield of produce of the host plant as compared to untreated plants; the lower concentrations and single dose of antipathogenic agents decrease the likelihood of damage to the plant or its crop as well as decrease the likelihood of any adverse side effects to workers applying the pesticide, or to animals or fowl which ingest the tissues or parts of treated plants.

Phytotoxicity of the formulation also is decreased when the antioxidant is eliminated. Reentry time to the greenhouse also is not an issue. Typically the formulations are rapidly lethal to a target organism; this is a particularly valuable characteristic when coupled with no reentry time, (for example, no loss of cut flower inventories).

For materials which are used as food stuffs for humans, or for feed for food commodities consumed by humans, there is an additional advantage in that toxins are decreased or eliminated in the food chain. As an example, the meat from fish, fowl and animals which have fed on materials such as contaminated grain or contaminated dried grasses also is contaminated by toxins. Although an aromatic aldehyde, cinnamic aldehyde, has been reported to exhibit antifungal properties, it has not previously been used on plants in a formulation which is intended to provide long-term protection to a plant so that mycotoxin contamination is avoided of consumables produced from the plant, not only fresh fruit or fresh vegetables, tobacco, and food grains for fowl, animals, fish, and other elements of the human food chain. Thus, treatment of the materials on which the animals feed decreases or eliminates toxins in the food chain. Another advantage of the invention is that environmental exposure of animals and humans to, for example, water and airborne sources of mycotoxins resulting from the burning and/or disposing of contaminated silage materials of all types is reduced by detoxification of the agricultural materials either before or after harvest by treatment to decrease or eliminate the fungi which produce the toxins. The detoxification procedures thus allow for the safe disposal of materials such as plants, plant materials and foodstuffs derived therefrom.

Compositions comprising natural products can be used to kill or displace toxin-producing organisms from the plants or plant parts that they colonize, thereby limiting the amount of toxin that normally accumulates on the material. The compositions are applied to the plant either before it is harvested or to a plant or other material after harvest and/or processing. Most preferably, the composition is applied to the plant, plant part or tissue before harvest. The composition is preferably biodegradable and most preferably is provided as an aqueous solution or as an emulsion in a biodegradable water-soluble anhydrous non-ionic surfactant, such as Tween 80, optionally together with a growth promotant and surfactant such as saponin, which can be derived from the *Yucca shidigera* plant. The susceptibility of particular fungi to the composition can be evaluated either *in vitro* or *in vivo*.

Of particular interest are various aldehydes, particularly aromatic aldehydes which can be used for direct killing of fungal pathogens and/or for the induction of systemic plant resistance to various fungal pathogens. The method includes the step of contacting and/or providing to one or more parts or tissues of a diseased plant or a plant susceptible to attack by pathogens with an antipathogenic agent in an amount sufficient to control growth of target pathogenic organisms. The growth modulating product includes natural compounds such as cinnamaldehyde, coniferyl aldehyde, and $\alpha$-hexyl cinnamic aldehyde to biocontrol pathogen infestations. By "biocontrol" is intended control of plant pathogens via direct antipathogenic activity and/or induced resistance of the host plant to pathogen infestation.

The method of the current invention is carried out by providing a fungus colonizing surface of a plant part such as a leaf, root, or flower part, or a tissue such as xylem or phloem, with a composition which includes as its active ingredient a natural product and/or the composition can be applied to the substrate in which the is growing or is to be

growing. The amount of antipathogenic agent that is applied either to the plant itself or to the rhizosphere will depend upon the degree of infestation and to some extent upon the formulation and the specific compounding used and therefore is empirically determined for best results. By "colonizing" is intended association of a microorganism or insect with a plant part or tissue from which the pathogen derives nutrients, typically essential nutrients such as amino acids. particularly methionine. By "natural product" is intended an organic compound of natural origin that is unique to one organism, or common to a small number of closely related organisms, and includes secondary metabolites of fungi and chemicals produced by plants. By "provided with" is intended external application to a plant part as well as induction of synthesis in the plant of antifungal compounds, either compounds endogenous to the plant and/or compounds supplied by genetic manipulation. Genetic manipulation can be performed by traditional cross-breeding methods or by introducing transgenes into the plant or an ancestor of the plant using recomoinant DNA technology. The natural products can be isolated from a natural source, be wholly or partially synthetic, or be produced by recombinant techniques either in the plant itself or in another organism. Examples of aromatic aldehydes of use in the present invention are cinnamic aldehyde :

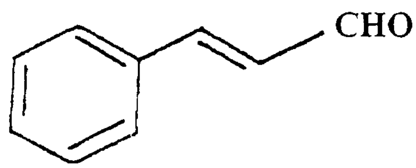

and coniferyl aldehyde :

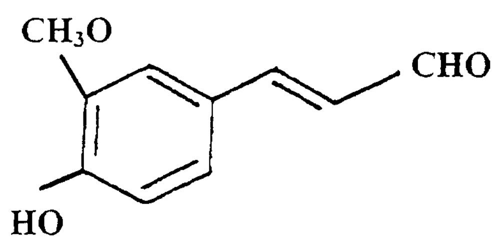

The chemical structure of alpha-hexylcinnamic aldehyde is shown in below.

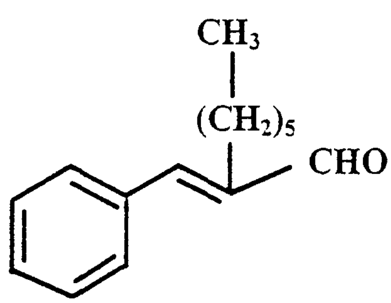

(5)

The Chemical Abstracts Service (CAS) name is 2-(phenylmethylene) octanal and the CAS Registry Number is [101-86-0]. The compound is also described by the chemical name of 2-hexyl-3-phenyl-2-propenal. The compounds's formula is $C_{15}H_{20}O$ and molecular weight is 216.3. HCA can be obtained from Firmenich; their product is composed principally of the (E)-cis isomer (93.8% maximum), and the (Z)-trans isomer (6% maximum). Among minor components is the self aldol condensation product of octanal (1-1.5% (Personal Communication, June Burkhardt, Firmenich, Plainsboro, New Jersey).

The compounds may be used either alone or in combination with other active or inactive substances and may be applied by spraying, pouring, dipping, in the form of concentrated liquids, solutions, suspensions, powders and the like. containing such concentration of the active compound as is more suited for a particular purpose at hand. They also can be applied, for example, in the form of dilute solution, in a suitable solvent directly to the rhizosphere either as part of an irrigation schedule or as a separate application.

For use as a foliar spray, although the aldehyde can be formulated alone, it can be rendered substantive by including a sufficient amount of an emulsifier such as Tween 80, or a compound such as saponin which has surfactant properties but does not significantly impact the antifungal properties of the formulation. Generally, detergents in the formulation do not detract from the antifungal properties of the aromatic aldehydes but do increase the substantive properties of the formulation. *See* for example. U.S. Patent No. 4,477.361. Other detergents which can be used include anionic detergents such as those described in U.S. Patent No. 4,978,686. Additional components such as an aqueous preparation of a salt of a polyprotic acid such as sodium bicarbonate, sodium sulfate, sodium phosphate or sodium biphosphate can be included in the formulation, to increase the antifungal properties of the formulation. The resulting emulsion is diluted to an appropriate concentration for use.

In a preferred embodiment, the formulation includes alpha hexyl cinnamic aldehyde, cinnamic aldehyde and/or coniferyl aldehyde in a formulation containing Tween 80 or saponin as an emulsifier and optionally sodium bicarbonate. The preferred formulation is an emulsion which contains alpha-hexyl cinnamic aldehyde, cinnamic aldehyde and/or coniferyl aldehyde, 0.5% to 10% by weight and may include the salt of an aprotic acid, 8% to 12% by weight and the balance water. Formulations with 6-12% of an aprotic acid are preferred. Generally, the total amount of aldehyde (s) present in the formulation is 5% or less. The formulations are effective without the use of antioxidants other than the inherent antioxidant properties of particular aldehydes, for example, coniferyl aldehyde.

Stability of the formulation can be evaluated by a variety of methods, including accelerated tests in which a formulation of interest is exposed to elevated temperatures over a set time. Samples of the formulations are taken at regular intervals and analyzed chemically by methods known to those skilled in the art to determine the rate and nature of degradation. For example, HCA can be analyzed by Gas Liquid Chromatography (GLC), using a 30 meter non-polar polydimethylsiloxane capillary column (e.g. HP-1, Hewlett-Packard, or SPB-1, Supelco) and a flame-ionization detector. Using helium as a carrier gas (8 ml/min.) and a column temperature of approximately 240°C, the (E)-cis isomer (major component) has a retention time of approximately 6.0 minutes and the (Z)-trans isomer (minor component) has a retention time of approximately 6.3 minutes.

The most effective antifungal amount for compositions including cinnamic aldehyde, coniferyl aldehyde and $\alpha$-hexyl cinnamic aldehyde which may find use can be determined using protocols known to those skilled in the art. As an example, for each particular application the mean disease resistance can be calculated; generally for effective pathogen control the mean percentage of disease control (MPDC) is greater than 60%, preferably at least about 70%. These protocols also can be used to optimize each formulation for specific pathogens using any of the compounds encompassed by formula (1). MPDC is defined by the formula:

$$\text{MPDC} = \frac{(\text{MDIC-MDIT})}{\text{MDIC}} \times 100$$

and

MDIC = Mean % of disease incidence in untreated controls
MDIT = Mean % of disease incidence in the treatment

The formulations also need to be evaluated for phytotoxicity; it therefore is important that at least one evaluation of the toxicity of the formulations be on living plants of the host variety. Phytotoxicity can be rated as follows in order of increasing severity of toxicity: 0-plants without any symptoms; 1-very slightly browning of hypocotyl (no other symptoms); 2-some wilting of plant, dying of lower leaves, some browning of vascular system; 3-wilting of entire plant, leaves dying, hypocotyl with external and internal symptoms; 4-necrosis of stem, plant dying. The phytotoxicity generally should be 2 or less, preferably 1 or less.

In some instances, the efficacy of the formulation can be increased by adding one or more other components to the formulation where it is desirable to alter particular aspects of the formulation. As an example, it may be desirable for certain applications to decrease the phytotoxicity effect when used pre-harvest or to increase the antipathogenic effect of the formulation or both. It is preferable that the other components minimize phytotoxicity while increasing the antipathogenic effect of the formulation. Of particular interest is the use of a component(s) to increase the mean disease resistance of a formulation against toxin producing microorganisms that are either colonized or capable of colonizing consumable product. The concentration of one or more of the other formulation ingredients can be modified to optimize

the antipathogenic and reduce the phytotoxic effect of the formulation. Of particular interest is the addition of a component to the formulation to allow for an overall reduction in the concentration of one or more other ingredients in a given formulation, particularly components according to formula (1), while maintaining overall efficacy of the formulation. Combination of such a component with other ingredients may be accomplished in one or more steps at any suitable stage of mixing and/or application.

Preferred additional components include saponins. Saponins are a class of compounds, each consisting of a sapogenin portion and a sugar moiety. The sapogenin may be a steroid or a triterpene and the sugar moiety may be glucose, galactose, a pentose, or a methylpentose. S. Budavari, ed., *The Merck Index,* 11th ed., Merck & Co., Inc., Rahway, N.J., 1990, p. 1328. The saponins for use in the present invention can be produced and/or isolated from various plant parts including fruit, leaf, seed and/or root, using means known in the art, from a variety of sources including the various plants known to produce them, ranging from yucca, quillaja, agave, tobacco, licorice, soybean, ginseng and asparagus to aloe woods. Saponins for use in with the present invention are preferably non-toxic to humans and higher animals. Most preferably the saponin for use in the present invention is non-toxic food grade, the source being from yucca plants. Even more preferred are the saponins from *Yucca schidigera* or *Y. valida* and their equivalents. The more preferred saponins for use in the present invention are derived from yucca plants, with the most preferred being *Yucca schidigera* or *Y. valida.*

A variety of structurally related saponins are known, the most variable structural feature being the glycosylation pattern. Saponins also may contain additional modifications, such as the sarasaponins which are saponins with a steroid attached, and saponin structure can be modified by any number of enzymatic, chemical and/or mechanical means known in the art. Saponins from *Yucca schidigera* contain steroidal saponins with the major sapogenins being sarsapogenin and tigogenin. The sarsaponin yields on hydrolysis, sarsasapogenin (sarsasapogenin 5-beta, 20-betaF, 22-deltaF, 25-betaF; also known as spirostan-3-beta-01 and parigenin), glucose and galactose. The sarasapogenim has a molecular formula of $C_{27}H_{44}O_3$. Nobel, Park S., *Agaves,* Oxford Univ. Press, New York, 1994.

Accordingly, derivatives of these compounds which produce a formulation having the desired antipathogenic and/or phytotoxic effect are considered equivalents of the invention. Depending on its structure, a given saponin can have a particular pesticidal property and lend use with the present formulations. Generally an effective amount of saponin is of the range of about 0.01 to 3% and most preferably about 0.25% v/v aqueous solution of 10° brix saponin extract. The brix degrees equals the percent by weight of sugar in the solution. Hawley, ed., The Condensed Chemical Dictionary, 10th ed., Van Nostrand Reinhold, New York, 1981, p. 149.

Each formulation is evaluated for its effect on specific toxin producing microorganisms and/or consumable product pre- or post-harvest such as a plant host or plant product using any of the aromatic aldehydes as well as other components of the formulation such as Tween 80 and/or sodium bicarbonate, with the combination and effective amount of each component adapted for a particular application to minimize toxicity while maintaining or increasing the antipathogenic effect of the formulation. The effective amount of each component may be determined by systematically varying the amount of that component in a test formulation, treating a crop of interest with the test formulation, and monitoring the mycotoxin levels in the plant host or plant product pre- and post-harvest and after a period of storage. An effective amount of a test component may be identified as the amount that controls the residual mycotoxin present in the plant product at a level acceptable for the particular crop of interest. For applications where the formulation is to be used to prepare the ground or other growth substrate for planting of host plants susceptible to particular pathogens, to apply to an already infested growth substrate, or to harvested material the formulations of the subject invention can be added directly to the rhizosphere, the substrate or the harvested material, or they can be bound to a solid support or encapsulated in a time release material. Where a solid carrier is used, materials which can lead to oxidation of the active aldehydes should be avoided. Examples of delivery systems include starch-dextran, and the like. *See* Yuan *et al., Fundamental and Applied Toxicology* (1993) 20: 83-87, for examples of delivery systems. Also *see* Kawada *et al.* (1994) 10: 385-389.

In addition to the specific aromatic aldehydes and optionally saponin as set forth above, derivatives of any of these compounds that produce a compound of the formula identified above upon action of a biological system on the derivative are considered to be equivalent to compounds of the invention. Thus application of precursor compounds to plant parts or tissues or harvested materials would be equivalent to the practice of the present invention. Biological conversion of precursor compounds into aromatic aldehydes is described in, for example, U.S. Patent Application No. 5,149,715 and references cited therein. *See* also Casey and Dobb *Enzyme Microb. Techol.* (1992) 14: 739-747. Examples of precursor compounds include those in pathways relating to acquired and/or systemic resistance to plant resistance to plant pathogens such as those in the amino acid ammonia lyase pathways, and include phenylalanine (to produce cinnamic acid). Other precursor compounds of interest include those in the biological pathways for the of lignins and coumarins, for example tyrosine to produce *p*-coumaric acid. Accordingly, precursors and derivatives of these compounds which produce a formulation having the desired antipathogenic and toxin reducing effect are considered equivalents of the invention.

Depending upon the target organism, the aldehyde to be used can be coupled to a solid support, optionally

through a linker such as a binding domain derived from a polysaccharidase, where the solid support is a polysaccharide such as cellulose, particularly microcrystalline cellulose. The preparation of cellulose binding domains is described in U.S. Patent Nos. 5,340,731; 5,202,247 and 5,166,317. The aldehydes can be coupled to the binding domains, with or without a cleavable bond, using methods well known to those skilled in the art. Binding domains from scaffold proteins also can be used. *See* Shoseyen *et al.* (PCT application PCT/US94/04132).

Other compounds can be used alone or in combination with the compositions to prevent the accumulation of toxins, and/or kill or displace one or more toxin producing microorganism, for example $H_2O_2$, which is known to kill particular fungi such as white rot fungus. Additionally, for use pre-harvest, compounds which induce non-systemic or systemic plant resistance to various fungi can be used to control colonization of certain fungi under field conditions. When a plant material is heavily contaminated with microbial toxin, for example, disposable or composted plant materials, the material additionally can be treated with an effective amount of an alkaline-peroxide or similar chemical which destroys or neutralizes the toxin present in the material. A mechanical process can be used separately or in combination with the chemical to promote the detoxification process. Examples of mechanical detoxification methods include any means that can be used to separate the toxin from the material or other means which destroys the toxin such as exposure to high heat and pressure.

If it is anticipated that a material may become contaminated with toxin, or if it is determined that the level of microbial toxin in a contaminated material makes the material unsuitable for safe consumption, the level of toxin can be controlled or reduced by treating the material so that the level of toxin is reduced to a level safe for consumption. More particularly, to reduce or counteract toxicity of microbial toxin-contaminated materials, an effective amount of one or more substances may be added to a product so as to counteract or preempt the toxic activity of the toxin. Effective substances include those containing thiols or sulfhydryl group amino acids, and sulfur amino acids for example cysteine, methionine and their derivatives. A preferred source of such amino acids can be chicken feather, hair, hides, hooves and other keratin animal products that have been treated so as to release the amino acids associated with them. Such treatment includes solubilization of keratin substrates in an alkaline-hydrogen peroxide (*e.g.*, NaOH + $H_2O_2$) environment. In a preferred embodiment, the alkaline/peroxide concentration is about 0.5-2.5%, but may be higher or lower as appropriate. Most preferably, the concentration of alkaline/peroxide is around 1-2%. Addition of sulfur amino acids to foods and feed counteracts the presence of mycotoxins, thereby reducing the potential health hazard associated with the consumption of these products.

The method of the present invention is carried out by introducing into a target pathogenic organism a sufficient amount of an antipathogenic agent to impair growth and/or viability of the target pathogenic organism. A formulation containing the antipathogenic agent is introduced to a plant tissue or part either pre- or post-harvest. For example, the formulation is sprayed on as a wet or dry formulation to the surface and/or underside of the leaves or other plant tissue or part of a plant infected with a plant pathogen, or of a plant susceptible to infestation with a plant pathogen, preferably to the point of run off when a wet formulation is used. The plants can be sprayed prior to or after infestation, preferably prior to infestation. However, in order to minimize damage to the host plant, where feasible, it is preferable to treat older plants, as young green leaves tend to be more susceptible to phytotoxicity. A plant growth promotant, such as saponin, is optionally used pre-harvest either in the antipathogenic formulation or as a separate formulation. Alternately, the formulation can be applied wet or dry to the rhizosphere where it can contact the roots and associated pathogenic organisms which colonize the roots. In some instances, time-release formulations may find use, particularly for applications to the rhizosphere, or to post-harvest materials.

The method of introducing the active ingredient(s) of the formulation into the target organism can be by direct ingestion by the pathogenic organism from a treated plant surface, or by feeding of a pathogenic organism on a nutrient-providing surface of a host entity, which is colonized by the target pathogenic organism, which host either contains or has on its surface the antipathogenic agent. The presence of the anti-pathogenic agent on a nutrient-providing surface of a host plant can be a result of direct contact of the antipathogenic agent with the plant part or it can be by elaboration from the host plant as a result of induction of systemic resistance as a secondary effect to prior treatment of the plant with the antipathogenic agent, or as a result of genetic modification of the host plant.

The aromatic aldehydes of the subject invention may be prepared by various synthetic methods known to those skilled in the art. For example, *see,* J. March, ed., Appendix B, *Advanced Organic Chemistry: Reactions, Mechanisms, and Structure,* 2nd Ed., McGraw-Hill, New York, 1977. Cinnamaldehyde may be prepared synthetically, for example, by oxidation of cinnamyl alcohol (Traynelis *et al., J. Am. Chem. Soc.* (1964) 86:298) or by condensation of styrene with formylmethylaniline (Brit. patent 504,125). The subject aldehydes may also be obtained by isolation from natural sources. For example, cinnamaldehyde may be isolated from woodrotting fungus, *Stereum subpileatum.* Birkinshaw *et al., Biochem. J.* (1957) 66:188.

HCA can be synthesized as described, for example, in USPN 5,055,621. On a laboratory scale, HCA can be synthesized by reaction of benzaldehyde with octanal under a nitrogen atmosphere (aldol condensation) (Personal Communication, Eric Walborsky, Firmenich Chemical Manufacturing Center, Port Newark, New Jersey). The reaction is conducted in a stirred flask charged with methanol, 309 ppm diphenylamine, potassium hydroxide and benzaldehyde.

Following the slow addition of octanal, the reaction mixture is brought to a pH of 7.5-9.5 with acetic acid. Following evaporation of methanol and wash of the reaction mixture with water, the organic phase is transferred to a distillation unit. Approximately 20-24% of the pot charge is removed as benzaldehyde and "lights", with the remaining distillate constituting alpha-hexylcinnamic aldehyde "heart cut." The "heart cut" is subjected to an additional fractionation, in which 1-5% (by weight) of the material may be removed in "light" fractions, depending upon odor evaluation. The commercial process differs from the laboratory scale procedure in that diphenylamine has been replaced by a proprietary catalyst[2]. The final product is a light yellow oil having a specific gravity of 0.955-0.965 at 20°C, a refractive index of 1.548-1.562 at 20°C, a boiling point of 305°C at 1 atmosphere, and a melting point of 26°C. The commercial product is stabilized with the addition of 0.04% 2, 6-di-tert-butyl-p-cresol (butylated hydroxytoluene or BHT), which serves as an anti-oxidant (Technical Data Sheet, Hexylcinnamic aldehyde 907600, Revision 853, Firmenich Inc., Plainsboro, New Jersey). HCA can also be isolated from rice where it has been reported to occur naturally. (Givaudan-Roure Index, Givaudan-Roure Corporation, Clifton, New Jersey, 1994, p. 89).

HCA is a low to moderately volatile compound, having a vapor pressure of 70 x $10^{-5}$ mm Hg at 25°C. Its parent compound, cinnamic aldehyde, has a vapor pressure approximately 40 times higher (2970 x $10^{-5}$ mm Hg at 25°C). For comparison purposes, the insect repellant N,N-diethyl-m-toluamine has a slightly higher vapor pressure (167 x $10^{-5}$ mm Hg at 25°C) (Reifenrath, W.G. (1995) *Volatile Substances. Cosmetics and Toiletries,* 110: 85-93).

Material in which the level of mycotoxin can be controlled can either be consumable or non-consumable and is preferably a plant or of plant origin, although any other material contaminated with fungi which produce microbial toxins or is capable of being colonized by or supporting the growth of toxin producing microorganisms can be treated. Of particular interest are crops intended for consumption by fowl, fish and animals, including humans, directly or indirectly. By "directly or indirectly" is intended that the crops could be ingested, for example, by humans (direct consumption), or that it is the nonhuman animal or fowl or fish which ingests the crop and is in turn ingested by humans (indirect consumption). Crops intended for consumption include tobacco, animal and fowl fodder, crops intended for processing into alcohol or food products such as corn syrup, and the like. Plants and plant materials colonized by toxin producing fungi include, for example, barley and other grasses, rice, corn, wheat, oats, hops, cassava, beans, potato, peanuts, sweet potato, tomato, sugar cane, coconut, citrus, grapes, sorghum, melons, cucumber, lettuce, spinach, artichoke, onion, tomato, strawberry and tobacco.

The level of mycotoxin also can be controlled in products derived from plant materials, such as processed juices, corn products such as high fructose corn syrup, oil, meal, starch, alcohol and products containing these and other corn derived ingredients and tobacco products such as cigars, cigarettes, and smokeless tobacco by inhibiting or preventing growth of toxin-producing fungi in the materials from which these items are produced either pre or post harvest. Similarly, microbial toxin levels also can ue controlled in forage grasses such as fescue, bent grass, alfalfa, clover, and turf grasses and in commercially prepared animal feeds including those for cattle, sheep, pigs, and horses; fowl such as turkeys and chickens; fish such as trout, catfish and salmon; domestic pet foods including dog and cat foods; and laboratory animal foods by treatment pre or post harvest of the materials themselves or precursor materials.

In addition to treating a host plant, seeds intended for consumption also can be treated using the subject formulations. The seeds can be dusted with a powder preparation (*see* U.S. Patent Application No. 4,978,686 for examples of inorganic materials to which the formulations can be adsorbed).

Risk exposure levels for various mycotoxins will vary depending on the use of the feed, food or tobacco. Different animals have different tolerances. Also the effects of the toxicity may be expected to appear only beyond the life expectancy of the animal or person. Adverse effects of contact with mycotoxins is minimized among field workers, researchers and test animals. In this way, the invention can be used to generally improve agricultural products so that they are safer for human, animal, fish or fowl consumption. By controlling microbial toxin levels associated with plants and products derived therefrom, animal and human health problems associated with the consumption of toxin may be reduced or eliminated.

The invention also finds use for reducing the adverse effects of chewing and smoking tobacco, as it is a theory of the invention that many tobacco-related diseases may result from the presence of mycotoxins in tobacco products. Thus, tobacco toxicity can be reduced by adding substances to reduce mycotoxins and other secondary metabolites, in particular by treating the tobacco with sufficient amounts of a pathogen-inhibiting composition to inhibit fungal metabolite production.

Similarly, the invention can be used to decrease mycotoxin contamination of evergreen and fruit trees, gross lumber, grapes, ornamental, grasses and contamination resulting from grafting of fruit trees. Further, prevention of such contamination allows plant derived foodstuffs or by-products materials to be disposed of in a manner that does not release toxins in a form that can become waterborne or airborne. Thus, the invention can be used to assist in the safe disposal of agricultural by-products such as rice, wheat and cotton hulls and plant debris in general, which are often disposed of by burning. Furthermore, the toxic contents of products made from plant materials known to be colonized by toxin producing microorganisms can be improved by determining the level of secondary metabolites produced by particular species of fungi, which colonize materials pre-harvest, for example, *Fusarium* species, and/or

post-harvest, for example, *Aspergillus* species. In particular, corn products like high fructose corn syrup can be improved by determining the level of certain toxins produced by *Fusarium* and then taking steps as necessary to reduce such levels.

Toxic secondary metabolites identified from organisms such as *Fusarium* spp. may also find use as pharmacological agents. It is to be anticipated that in this area, the recognition of previously unappreciated chemical structures will yield a number of structures for research as to their properties as herbicides, insecticides, fungicides, and pharmacological agents and for research into methods for producing the structures in commercial amounts.

The following examples are offered by way of illustration and not by way of limitation.

## EXAMPLES

### Example 1

### Treatment of Fungal Pathogens on Corn

A three-treatment experiment with cinnamic aldehyde formula and components, coniferyl aldehyde formula and combined cinnamic and coniferyl aldehyde formulas is evaluated on field grown corn known to be susceptible to pathogenic fungal infestation. The plants are blocked by variety before fungicide treatments and are randomized as to the plants. Various varieties susceptible to fungal infestation are tested using the following protocol which evaluates the effect of cinnamic aldehyde and/or coniferyl aldehyde alone and in combination with Tween 80 and/or $NaHCO_3$.

Each plant receives a single foliar spray to run off following evaluation of fungal infection (after Paulus and Nelson (1988) *Calif. Agric.* 42:15). The response variable recorded for each plant is the fungal infection rating based on the Paulus/Nelson (*supra*) rating scale. Plants are evaluated on this scale just prior to and four days after treatment.

### Example 2

### Treatment of Pitch Canker Disease

### (*Fusarium subglutinans*)

#### (a) *In vitro* test

Pitch canker disease, caused by the fungus *Fusarium subglutinans* f.sp. pini is characterized by a resinous exudation on the surface of shoots, branches, exposed roots and boles of infested trees. The host and geographic range of the pitch canker pathogen has greatly increased since it was first discovered in California in 1986. The pathogen has recently been discovered in Mexico and Japan. An association of Engraver beetles (Scolytidae; IPS species) as vectors of the Pitch Canker Fungus has been proposed by Fox *et al.* (1991) *Plant Dis.* 75:676-682).

A bioassay based on inhibition of radial growth of *Fusarium subglutinans* f. sp. pini was used to test various aldehyde formulations. The bioassay was performed in a double blind fashion under sterile conditions. All concentrations given are those of particular solutions before dilution in the agar. Eight ml of test formulation was pipetted into 200 ml of molten 2% potato dextrose agar (DIFCO), and the mixture was dispersed into 5 plastic petri dishes (25 ml dish). Test formulations of 5 ppm benomyl were used as a positive control and sterile $H_2O$ as a negative control. Each of four plates was inoculated at the center with an agar plug transferred from a growing PDA culture of *Fusarium subglutinans* f. sp. pini (isolate SL-1, UCB) for each test formulation. A fifth plate was left noninoculated as a control for inhibited growth.

All inoculated and non-inoculated plates were incubated at 18° C for 5 days, after which colony diameters were measured. The results were provided in the Table below. The larger the colony, the less effective the test formulation. No growth of the colony (diameter=0) indicates a maximum growth inhibition.

The results show that concentrations of cinnamic aldehyde in vehicle (with or without the addition of saponin) inhibited radical growth of *Fusarium subglutinans,* as did 2% glutaraldehyde. Radial growth was completely inhibited at 12,500 ppm. (*See* Table 1.) The increase in radial growth seen with the addition of saponin may be due to the growth promotant characteristics which have been reported for saponin.

#### (b) Treatment of Monterey Pine Seeds

Seeds of Monterey Pine, half of which are dusted with a starch-dexta formulation containing cinnamic aldehyde and/or coniferyl aldehyde, 10 to 1000 ppm, are planted in vermiculite into which has been admixed a formulation of cinnamic aldehyde and/or coniferyl aldehyde, 10 to 1000 ppm. An inoculum of *Fusarium* which causes pine canker

disease is added to the vermiculite at the time the seeds are planted. Seed in untreated vermiculite are used as a control.

Example 3

Treatment of Strawberry Red Core

(*Phytophthora Fragariae*)

Strawberry red core disease is caused by the fungus *Phytophthora fragariae* Hickman which is spread by means of infected planting material or soil infested with long-lived oospores of infected debris. Various formulations containing cinnamic aldehyde and/or coniferyl aldehyde are tested as follows: Macerated strawberry roots infected with *Phytophthora fragariae* are thoroughly mixed with infested compost and allowed to decompose for 4 to 6 weeks to produce a well rotted inoculum for treatment. This is divided into 1 kg lots and mixed with 1500 ml of a test formulation at different concentrations (*see* Table 2). After 10 minutes treatment, the compost is rinsed under running tap water on a 25 mm sieve for a minimum of 5 minutes to remove all traces of the test formulation. The compost is then put into 9-cm diameter plastic pots and planted with 4 strawberry plants per pot. Five pots are used for each treatment. Plants are grown in a controlled environment room at 15°C and 18-h daylength; the compost is kept damp to encourage infection. Pots are placed on grids to avoid cross infection among treatments.

After 9 weeks the strawberry plant roots are washed free of compost and examined for signs of infection by cutting roots longitudinally and looking for red steles, and rotted or brown roots. All infections are confirmed by microscope examination of root pieces for the presence of oospores of *Phytophtora fragariae.*

Table 1

Effect of Aldehydes on Radical Growth of *Fusarium subglutinas* f.sp. *pini*

| | Cinnamic Aldehyde[1] (ppm)[2] | | | | | | |
|---|---|---|---|---|---|---|---|
| | None | 10 | 100 | 2,500 | 5000 | 12,500 | 25,000 |
| None | 4.038 | 4.363 | 4.238 | 3.513 | 2.980 | 0 | 0 |
| Saponin[3] | 4.138 | 4.088 | 4.300 | 3.600 | 2.913 | 0 | 0 |
| Benomyl[4] | 0 | | | | | | |
| Tween 80 (2%) + $NaHCO_3$ (6%) | 4.380 | | | | | | |
| Glutaraldehyde[5] | 3.663 | | | | | | |
| $H_2O$ | 3.738 | | | | | | |

[1] All cinnamic aldehyde formulations are made up in 2% Tween 80, 6% $NaHCO_3$

Table 2

Treatment Protocol

| Group | Treatment | Ingredient(s) | Amount of treatment ingredient(s) (balance $H_2O$ to 1000g) |
|---|---|---|---|
| 1 | A | Cinnamic aldehyde | 5g |
| 1 | B | Tween 80 (T80) | 10g |
| 1 | C | $NaHCO_3$ | 80g |
| 1 | D | Cinnamic aldehyde+T80 | 5g, 10g |
| 1 | E | Cinnamic aldehyde+$NaHCO_2$ | 5g, 80g |
| 1 | F | $NaHCO_3$+T80 | 80g, 10g |
| 1 | G | Test formulation 1 | A=5, B=10g, C=80g |
| 1,2,3 | H | +Control (Benomyl) | per manufacture instructions |
| 1,2,3 | I | -Control | no treatment |
| 2 | J | Coniferyl aldehyde (COFA) | 5g |
| 2 | K | COFA+T80 | 5g, 10g |
| 2 | L | COFA+$NaHCO_3$ | 5g, 80g |
| 2 | M | Formula 2 | J=5g, B=10g, C=80g |
| 3 | N | Cinnamic aldehyde+COFA | 2.5g, 2.5g |

Table 2
(continued)

| Group | Treatment | Ingredient(s) | Amount of treatment ingredient(s) (balance $H_2O$ to 1000g) |
|---|---|---|---|
| 3 | O | Cinnamic aldehyde+COFA+T80 | 2.5g, 2.5g, 10g |
| 3 | P | Cinnamic aldehyde+COFA+$NaHco_3$ | 2.5g, 2.5g, 80g |
| 3 | Q | Formula 3 | A=2.5g, J=2.5g, B=10g, C=80g |

Example 4

Post-Harvest Handling of Citrus Fruit

The purpose of this experiment is to evaluate the citrus fruit post-harvest preservative activity of cinnamic aldehyde. Forty California oranges are selected at random from a packing house lot after elimination of freeze-damaged fruit. Twenty treatment oranges are treated in a tank with soap and concentration of test formulation, then washed and brushed with soap and biocide. After 5 minutes, treated citrus are rinsed in fresh water. Controls are only washed with fresh water. Both treated and untreated lots are dried (water eliminated from fruit surface). The treated lot is sprayed in tumbler with 100 ml of formulation, air dried 10 minutes, packed in carton and placed in temporary storage. The control lot is sprayed in tumbler with 100 ml of distilled $H_2O$, air dried 10 minutes, packed in carton and placed in temporary storage. After twenty days both lots are evaluated for physiological and pathological breakdown.

Example 5

Post-Harvest Handling of Underground

Vegetables (Roots, Tubers and Bulbs)

The purpose of this experiment is to evaluate the underground vegetable preservative activity of cinnamic aldehyde. The edible portions of this group of vegetables develop mostly underground and include several botanical structures.

- Roots: beet, carrot, celeriac, radish, horseradish, parsnip, sweet potato, cassava, jicama;
- Tubers: potato, Jerusalem artichoke, yam;
- Bulbs: onion, garlic, shallot.

Method

Forty tuber potatoes are selected at random from field bins. Potatoes are cured in a packinghouse for six days to insure formation of periderm. Potatoes are removed from curing and into two groups of 20 each for trial. Twenty potatoes in treated group are washed, then sprayed with 10 ml each of formulation. Untreated potatoes are washed with clean $H_2O$. Treated and untreated potatoes are packed separately in consumer package and put in temporary storage. Potatoes are observed and evaluated at intervals of 30, 60, 90 and 120 days for pathological breakdown.

Example 6

Post-Harvest Methods for Fruit Vegetable Handling

The aim of this experiment is to evaluate the fruit vegetable preservative activity of cinnamic aldehyde. Fruit vegetables are not generally adaptable to long-term storage. Exceptions are hard-rind (winter) squashes and pumpkin. Immature fruit vegetables of concern are the legumes, curcurbits (soft rind squash) solanaceous vegetables (eggplant, peppers, *etc.),* okra and sweet corn. Mature fruit vegetables of interest are curcurbits (cantaloupe, honeydew or other muskmelons; watermelon, hard-rind squashes and pumpkins). (Solanaceous vegetables: mature green and vine ripe tomatoes, ripe peppers.)

Method

Forty fruit vegetable tomatoes (vine ripe) are selected at random from bulk gondolas at a packing house. Twenty treated tomatoes are clear water rinsed, then treated with spray of 100 ml of test formulation on roller conveyer section. Twenty untreated tomatoes are sprayed with 100 ml $H_2O$ on roller conveyor sections. Treated and untreated are put in separate place pack (treated and untreated) and stored in temporary storage for 10 days. Tomatoes are observed and evaluated on days 3, 6 and 10 for pathological breakdown.

Example 7

Preservative Activity of Cinnamic Aldehyde

The aim of this experiment is to evaluate the flower, leafy and stem vegetable preservative activity of cinnamic aldehyde. The leafy, stem and floral vegetables are represented by (not limited to) the following commodities:

- Leafy vegetables: lettuce, cabbage, Brussel sprouts, celery, spinach, green onions, Witloaf chicory, endive;
- Stem vegetables: asparagus, kohlrabi, fennel;
- Floral vegetables: artichoke, broccoli, cauliflower

Method (Post-Harvest Example of Floral Vegetable: Cauliflower)

Forty heads of cauliflower are selected from a packinghouse lot. Leaves are trimmed from cauliflower. Twenty treated heads are sprayed with 10 ml test formulation each and the heads wrapped. Twenty untreated heads are sprayed with 10 ml $H_2O$ and then wrapped. Treated and untreated heads are put in separate boxes and stored in temporary storage for 10 days. Heads are observed on days 3, 6 and 10 for pathological breakdown.

Example 8

Post-Harvest Treatment of Cut Flowers

Rose Water Solution Microbes

Long life of cut flowers depends on maintenance of sufficient water status of the cut flowers from the moment of harvest until the flower fades in the home. The two critical factors to good water status are rehydration after dry handling, and control of vase solution bacteria whenever the stems are in water. Although a range of commercial biocides are used in vase preservatives for cut flowers, none is particularly effective or long lasting. This bioassay tests the possibility of preventing the growth of bacteria in vase solutions by using a cinnamic aldehyde formulation which has the potential for a new type of vase preservative and cut flower vase solution biocide.

Methods

Twenty fresh decapitated roses (*Hybrid tea*) and twenty fresh decapitated carnations (*Dianthus caryophyllus*) are cut so that 4 cm of stem extends beyond the ring top of a plastic baby nursing bottle. Flowers are each placed in separate bottles secured to bench with tape and lined with sterile baby bottle liners. Ten of each type of cut flowers serve as treatment group, ten as control. Both flower groups are given 10 ml $H_2O$. Treated cut flowers receive range of formulation from 20-50 ppm. Senescence of cut flowers is observed and recorded.

Example 9

Treatment of Late Blight (*Phytophtora infestans*)

Late blight affects tomato. potato, eggplant and other potato family plants: it begins when fungal spores settle on wet plant surfaces during periods of mild temperature. Experiments to test for control of *Phytophtora infesrans* are conducted using potato seedlings in the greenhouse. Plants are spray-inoculated with using an isolate of the pathogen in the greenhouse to achieve. Plants are treated either prior to or after the inoculation with the pathogen using a treatment protocol such as that shown in Table 2. including a third panel for testing alpha-hexyl cinnamic aldehyde. An additional treatment regimen using a sufficient amount of saponin in place of the Tween as an emulsifier is also tested. Treatment effects are evaluated at two and three weeks post-treatment.

As most food for human and animal consumption is of animal or plant origin, and given the known activity of microbial toxins, the presence of microbially produced toxins that accumulate before harvest and or after harvest and persist after processing are likely to be at least partially responsible for a multitude of health disorders linked to mycotoxin contamination. By controlling microbial toxin levels in consumable products and those found

As most food for human and animal consumption is of animal or plant origin, and given the known activity of microbial toxins, the presence of microbially produced toxins that accumulate before harvest and or after harvest and persist after processing are likely to be at least partially responsible for a multitude of health disorders linked to mycotoxin contamination. By controlling microbial toxin levels in consumable products and those found associated with

certain materials found in the environment, cancer, liver disease and other diseases linked to microbial toxin ingestion may be reduced, particularly those associated with the consumption of contaminated cereal grain foods and animal feeds and those associated with processed grain products such as corn syrup. In addition, laboratory animal testing procedures may also be improved by controlling the mycotoxins associated with the animal foods and certain test materials such as tobacco. Moreover, by controlling the level of microbial toxin associated with tobacco, cancers in humans linked to tobacco chewing or smoking may be reduced.

Toxic metabolites associated with materials of interest include those produced by bacteria and or fungi, and especially fungal mycotoxins and other secondary metabolites having similar properties. The methods and compositions of the invention are particularly suited to control the level of one or more mycotoxins produced by fungi from the genera *Aspergillus, Alternaria, Fusarium,* and *Penicillium,* and more particularly those fungi which produce one or more mycotoxins including aflatoxins, fumonisins, fusaric acid, TA/AAL toxins, zearalenone, and trichothecene, 5-butylpicolinic acid and related phytotoxic pyridine derivatives.

## Claims

1. A method for biocontrol of toxin-producing microorganisms on a harvested plant part, said method comprising: providing a plant part prior to or after harvesting of said plant part with an effective microbial growth modulating amount of a formulation comprising saponin or polyoxyethylene(20)sorbitan mono oleate and 0.01 g/l to 25 g/l of at least one of cinnamic aldehyde, coniferyl aldehyde or α-hexyl cinnamic aldehyde, wherein said formulation is non-phytotoxic to said plant and no other antioxidants than cinnamic aldehyde, coniferyl aldehyde or α-hexyl cinnamic aldehyde are provided; whereby microorganisms on said plant part are biocontrolled.

2. The method according to claim 1, wherein said growth modulating amount provides a mean disease resistance of about 70% or higher.

3. The method according to any of claims 1 or 2, wherein said providing is prior to harvesting of said plant part.

4. A method according to any one of claims 1-3, wherein inhibition of growth of said micro-organism is for a period greater than one month.

5. Harvested plant or plant parts substantially free of fungi obtained according to the method of any one of the claims 1-4.

## Patentansprüche

1. Verfahren zur biologischen Bekämpfung von toxin-erzeugenden Mikroorganismen auf einem geernteten Pflanzenteil, wobei das Verfahren aufweist: Ausstatten eines Pflanzenteils vor oder nach dem Ernten des Pflanzenteils mit einer effektiven, das mikrobielle Wachstum modulierenden Menge einer Formulierung, die Saponin oder Polyoxyethylen(20)sorbitanmonooleat und 0,01 g/l bis 25 g/l von mindestens einer Substanz von Zimtaldehyd, Coniferylaldehyd oder α-Hexylzimtaldehyd aufweist, wobei die Formulierung nichtphytotoxisch für die Pflanze ist und keine anderen Antioxidanzien als Zimtaldehyd, Coniferylaldehyd oder α-Hexylzimtaldehyd vorgesehen sind, wodurch Mikroorganismen auf dem Pflanzenteil biologisch bekämpft werden.

2. Verfahren nach Anspruch 1, bei dem die das Wachstum modulierende Menge eine mittlere Krankheitsresistenz von ungefähr 70% oder höher vorsieht.

3. Verfahren nach Anspruch 1 oder 2, bei dem das Ausstatten vor dem Ernten des Pflanzenteils stattfindet.

4. Verfahren nach einem der Ansprüche 1 bis 3, bei dem die Verzögerung des Wachstums des Mikroorganismus für eine Zeitdauer größer als einen Monat anhält.

5. Geerntete Pflanze oder Pflanzenteile, die im wesentlichen frei von Pilzen sind und durch das Verfahren nach einem der Ansprüche 1 bis 4 erhalten sind.

## Revendications

1. Procédé de biocontrôle des microorganismes producteurs de toxines sur une partie d'une plante récoltée, ledit procédé comprenant : l'introduction, dans une partie de plante, avant ou après la récolte de ladite partie de plante, d'une quantité efficace de modulation de prolifération microbienne d'une formulation comprenant de la saponine ou du mono-oléate polyoxyéthylènique(20) de sorbitanne et de 0,01 g/l à 25 g/l d'au moins l'un de : l'aldéhyde cinnamique, l'aldéhyde coniférylique ou l'aldéhyde cinnamique d'α-héxyle, dans lequel ladite formulation est non-phytotoxique pour ladite plante, et dans lequel aucun autre antioxydant que l'aldéhyde cinnamique, l'aldéhyde coniférylique ou l'aldéhyde cinnamique d'α-héxyle n'est introduit ; et grâce à ce procédé les microorganismes sur ladite partie de plante sont biocontrôlés.

2. Procédé selon la revendication 1, dans lequel ladite quantité de modulation de prolifération procure une résistance moyenne à la maladie d'au moins environ 70 %.

3. Procédé selon l'une quelconque des revendications 1 ou 2, dans lequel ladite introduction précède la récolte de ladite partie de plante.

4. Procédé selon l'une quelconque des revendications 1 à 3, dans lequel l'inhibition de la prolifération dudit microorganisme dure plus d'un mois.

5. Plante ou parties de plante récoltée(s) sensiblement exempte(s) de champignons obtenue(s) selon le procédé de l'une quelconque des revendications 1 à 4.